# EUROPEAN PATENT APPLICATION

(11) **EP 0 719 547 A1**
(43) Date of publication of application: **03.07.1996**
(21) Application number: 94922270.7
(22) Date of filing: 18.07.1994
(51) Int. Cl.: A61K 9/16, A61K 9/20

(54) **SELF-DESINTEGRATING COMPACT GRANULES USED IN GALENIC FORMS AND CONTAINING ACTIVE INSOLUBLE OR HARDLY SOLUBLE PRINCIPLES**

(71) Applicant: Carvajal Martin, Luis Cipriano, E-45005 Toledo (ES); Ledger, Ian, Anthony, David, B-1401 Baulers (BE)
(72) Inventor: Carvajal Martin, Luis Cipriano, E-45005 Toledo (ES); Ledger, Ian, Anthony, David, B-1401 Baulers (BE)
(74) Representative: Richebourg, Michel François
(86) International application number: ES9400073
(87) International publication number: WO9602234

(57) **Abstract**

Self-desintegrating compact granules used in galenic forms and containing insoluble or hardly soluble active principles, such as for example in tablets, capsules, bags or extemporaneous mixtures, characterized essentially in that they are comprised of an intimate and homogeneous mixture of finally divided active principle, an intumescent hygroscopic agent and an emulgent.

## Description

The subject of the present invention as herein described consists of COMPACT AUTODISINTEGRATING GRANULES FOR USE IN GALENIC FORMS CONTAINING INSOLUBLE OR POORLY SOLUBLE ACTIVE PRINCIPLES
The compact granules, subject of this patent, contain a mixture of three components - the Active Principle which, in order to exercise its therapeutic action, must be accessible to the biological fluids which aid its absorption by the mucus membranes of the body, the tensioactive, which has the property of facilitating suspension of the particles of the Active Principle in water, and finally, a hygroscopic intumesceent agent which in contact with water greatly increases in volume so that it breaks up and disintegrates the granule or tablet.

The habitual process of creating a galenic form consists in milling the Active Principle, adding an appropriate quantity of excipient, mixing and compacting. The process used up until now has serious disadvantages with respect to the utilisation of pharmaceutical products and the efficacy of disintegration and bioavailability of the Active Principle when this is insoluble or sparingly soluble.

Current practice in the pharmaceutical industry is to produce granules which are made into tablets or capsules. When the Active Principles are insoluble or sparingly soluble in water, the disintegration of these granules and the liberation of the Active Principle to be absorbed in biological fluids is difficult for the following reasons:
Sometimes gels are formed on the surface of the granule impeding the penetration of solvent.

If the Active Principle is very sparingly soluble in water, the gastric juices do not "wet" the granule containing the Active Principle and there is no solubility.

If the granules have a slow speed of disintegration a part of these are not disintegrated during passage through the stomach, with the result that an orally administered Active Principle can be eliminated to a significant degree without being absorbed by biological fluids, which signifies a much lower bioavailability than that intended in the therapeutic dose.

If, on the contrary, the Active Principles are administered in finely divided powder, there are considerable difficulties in dose division and powder handling.

In practice the result can be a lower certainty that the administered dose produces uniform, repetitive and safe levels in the blood and biological liquids, which are therapeutically necessary, because it is not possible to completely rely on the physical process of the disintegration and liberation of the Active Principle. This is of considerable importance in insoluble or sparingly soluble substances where absorption is limited by the direct relationship with the liberation of the Active Principle. In current practice attempts are made to avoid these difficulties by using a higher weight of Active Principle than the therapeutic need in order to compensate for losses, with the danger that there could be individual cases in which a combination of factors provokes biological access of more Active Principle than required provoking an uncontrolled overdose, or that only the correct therapeutic dose is used with the danger that the curative action desired is not realised due to the fact that the organism does not have access to the required quantity of Active Principle, or that the galenic forms created for these Active Principles contain a large quantity of excipients, which in the case of high therapeutic doses in tablet form, their size can increase to that of the human trachea so that the patient has to swallow a tablet of inconvenient and sometimes dangerous volume.

The COMPACT AUTODISINTEGRATING GRANULES FOR USE IN GALENIC FORMS CONTAINING INSOLUBLE OR POORLY SOLUBLE ACTIVE PRINCIPLES, object of the present invention substantially improves the galenic formulations which have been used to date. The granules are formed by the roller compaction of a mix of the finely divided Active Principle, the hygroscopic intumescent agent, the tensioactive and other biologically inactive excipients which are used in conventional formulations. The small particle size of the Active Principle which is normally the case when it is insoluble or sparingly soluble, can present the difficulties mentioned above. The hygroscopic intumescent agent provides physical properties at the particulate level which signify an important innovation in current galenic formulations by rapidly absorbing water molecules and violently increasing in volume, neutralising and breaking the cohesive forces and mechanical bonds which maintain the particles of the tablet together at a much faster rate than is habitual. The tensioactive ensures that the fine particles of the insoluble or sparingly soluble Active Principle do not form gels and are maintained in suspension thus conserving the original fine particle size.

This interesting innovation in the structure of a galenic form offers unlimited improvements in different pharmaceutical products to be presented to the market. The granules manufactured under the current invention avoid and counter the disadvantages referred to above and can be used to great advantage in pharmaceutical forms such as swallow, dispersible or chewable tablets of significantly smaller size than those habitually on the market. The size reduction is due to the lack of the necessity to use large quantities of excipients in order to avoid the formation of gels of the Active Principle during a slow disintegration of the galenic form either in biological fluids or in water.

The low content of excipients required for rapid disintegration in the present invention, considerably reduce the volume of the mix so that the granules can be used in capsules.

Due to the property of the granules which disintegrate to the original particle size of the milled Active Principle, the following advantages are obtained in their use and manipulation.

A significant improvement in the mechanical behaviour of the initial mix.

A reduction (almost removal) of inert excipients which are used to counteract gels formed by slow biological disintegration.

An improvement in weight control.

In extemporaneous presentations the following advantages may be additionally obtained:
Improvement in flow properties and the reduction of container sizes.

Given that granules of the galenic form which is the subject of the present invention can be used in different pharmaceutical products, there is an improvement in factory management by reducing the complexity of the machines and reducing or eliminating certain intermediate stocks and other logistical complications.

Some illustrative examples which are not limitative for formulations of highly self-disintegrating granules with different Active Principles are:

### Example No 1

| Raw materials | Quantity (mg) | Function |
|---|---|---|
| Ampicillin | 500 | Active Principle |
| Polyplasdone XL | 17 | Disintegrant |
| Dioctylsulfosuccinate sodium | 2.5 | Tensioactive |

### Example No 2

| Raw materials | Quantity (mg) | Function |
|---|---|---|
| Acetylsalicylic acid | 500 | Active Principle |
| Polyplasdone XL | 20 | Disintegrant |
| Sucrosester | 2 | Tensioactive |

### Example No 3

| Raw materials | Quantity (mg) | Function |
|---|---|---|
| Paracetamol | 500 | Active Principle |
| Polyplasdone XL | 15 | Disintegrant |
| Dioctylsulfosuccinate sodium | 2 | Tensioactive |

Other disintegrants can be used, for example sodium starch glycollate (Primogel) or croscarmellose sodium. Other tensioactives can be used such as other salts of dioctylsulfosuccinic acid or various sucrose esters or different types of Tweens. The typical quantities of tensioactives are between 0.3% and 0.5% depending on the insolubility of the Active Principle or its capacity to form gels or of its wetting properties. The habitual quantities of disintegrants are between 1% and 6% depending on the capacity of their plastics deformation and their increase in volume on water absorption.

Measures of the granulometry of the above formulations gave the following results:

| Milled Active Principle | |
|---|---|
| Particle Size | % |
| >200 µ | 1-5 |
| >100 µ | 20-25 |
| > 50 µ | 12-15 |
| < 50 µ | > 50 |
| When compacted and granulated the following granulometry is obtained: | |
| >600 µ | 29-32 |
| >500 µ | 14-16 |
| >400 µ | 12-14 |
| >300 µ | 10-12 |
| >200 µ | 6-8 |
| >100 µ | 16-22 |
| <100 µ | 4-6 |
| The granulometry of the suspension obtained from the autodisintegrating granule in water is as follows: | |
| >200 µ | 5-7 |
| >100 µ | 20-25 |
| > 50 µ | 12-15 |
| < 50 µ | > 50 |

It can be seen that following autodisintegration in water the particle size is practically identical to that of the milled product.

Another non-limitative example of a formulation for tablets or sachets where the tablets have the property of being swallowable or dispersible or chewable is:

### Example 4

| | |
|---|---|
| Autodisintegrating granule | 519.5 mg |
| Polyplasdone XL | 5.0 mg |
| Aspartame | 10.0 mg |
| Flavour | 4.0 mg |
| Magnesium stearate | 1.2 mg |

The total quantity of excipients required to produce an autodisintegrating tablet is less than 10% of the quantity of Active Principle (8% in the example).

A non-limitative example of the autodisintegrating granule for capsule filling would be:

### Example 5

| | |
|---|---|
| Autodisintegrating granule | 519 mg |
| Magnesium stearate | 1.2 mg |

As can be observed, the basis of the formulation in these examples is the autodisintegrating granule and manufacture can be simplified in a manner analogous to a tree where the trunk is the manufacture of the granule and the branches are the manufacture of distinct galenic presentations.

With the objective of illustrating further these ideas a schematic representation is attached and forms an integral part of this descriptive memorandum. This is only illustrative and not limitative as to the practical possibilities of the invention.

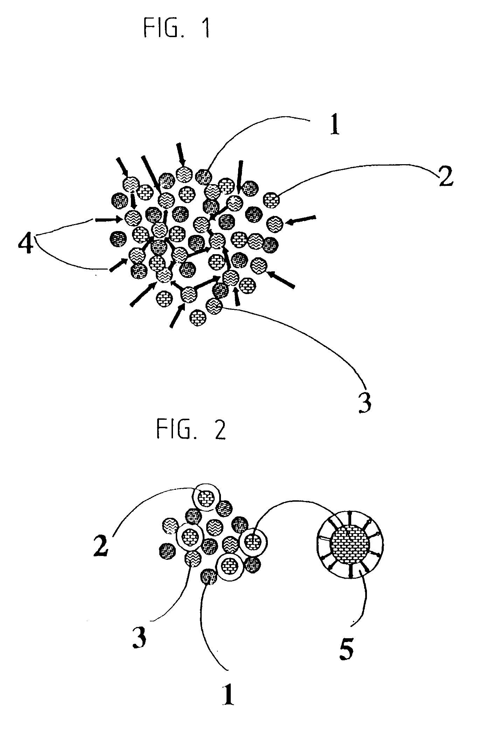

Figure 1 of these diagrams shows the function of the tensioactives in the granules and Figure 2 shows the action of the hygroscopic intumescent agent once an intimate mixture is obtained.

In accordance with the diagrams and their numeration, THE COMPACT AUTODISINTEGRATING GRANULES FOR USE IN GALENIC FORMS CONTAINING INSOLUBLE OR POORLY SOLUBLE ACTIVE PRINCIPLES are composed of three intimately mixed ingredients. The Active Principle - 1 -, the tensioactive - 2 -, and the disintegrant - 3 -. The ingredients have to be blended to form a uniform mixture. When powders are of fine particle size the specific surface of the material is increased and when the mixture is compacted a material of homogenous aspect is obtained. The increase of the specific surface and the uniform mixture gives the materials multiple channels -4- which conduct water to the hygroscopic intumescent agent when the tablet or granule is in contact with water. When this occurs the particles of the hygroscopic intumescent agent increase in volume -5- breaking up the compacted material. There is a double mechanism which disintegrates the material more quickly which significantly improves the bioavailability of the Active Principle.

The preceding description covers the fundamental principles of the idea and small details may be changed in its application.

## Claims

1. COMPACT AUTODISINTEGRATING GRANULES FOR USE IN GALENIC FORMS CONTAINING INSOLUBLE OR POORLY SOLUBLE ACTIVE PRINCIPLES made up by a homogeneous mixture of the active principle reduced to very small solid particles, a hygroscopic intumescent agent, and a tensioactive agent.
